# EUROPÄISCHE PATENTSCHRIFT

(11) **EP 2 711 049 B1**
(45) Veröffentlichungstag und Bekanntmachung des Hinweises auf die Patenterteilung: **01.03.2017**
(21) Anmeldenummer: 13183498.8
(22) Anmeldetag: 09.09.2013
(51) Int. Cl.: A46B 9/04, A46B 15/00, A46B 11/00, A61Q 11/00, A46D 1/00

(54) **Zahnbürste**
Toothbrush
Brosse à dents

(30) Priorität: 25.09.2012 CH 17332012
(43) Veröffentlichungstag der Anmeldung: 26.03.2014
(73) Patentinhaber: megasmile AG, 9053 Teufen (CH)
(72) Erfinder: Zettel, Roland, 9053 Teufen (CH)
(74) Vertreter: Schneider Feldmann AG Patent- und Markenanwälte

(56) Entgegenhaltungen:
- WO-A1-98/55001
- US-A1- 2009 092 563

## Beschreibung

### Technisches Gebiet

Die vorliegende Erfindung beschreibt eine Zahnbürste umfassend Borsten aus Kunstfasern und ein Zahnreinigungssystem zur Reinigung von Zahnoberflächen von Patienten mit Orthodental-Brackets mit einer Zahnbürste.

### Stand der Technik

Zahnpasta, Zahnpasten oder Zahncremes werden seit geraumer Zeit zur verbesserten mechanischen Zahnreinigung unter Verwendung von Zahnbürsten in jeweils verschiedenen Ausführungsformen verwendet.

Neben Wirkstoffen zur Kariesprophylaxe umfassen handelsübliche Zahnpasten eine Reihe unterschiedlicher Substanzen, beispielsweise Aromastoffe, Konservierungsmittel, Schaumbildner und Feuchthaltemittel, aus denen eine weiche Zahnpaste mit ansprechendem Aussehen und cremiger Konsistenz gebildet ist.

Zur Unterstützung der mechanischen Reinigung der Zahnoberflächen zur Beseitigung von Plaque und Bakterien enthalten die heutigen Zahnpasten einen Putzkörper umfassend Abrasivstoffe. Diese Abrasivstoffe umfassen üblicherweise mineralische Stoffe in unterschiedlichen Körnungen, welche abrasive Wirkungen aufweisen, womit die Zahnoberflächen schonend gesäubert werden können.

Bei Patienten mit Orthodentalapparaturen, insbesondere Brackets, kommt es bei der Zahnreinigung mit handelsüblichen Zahnpasten immer wieder zu störenden Ablagerungen von Zahnpastenrückständen aufgrund der Abrasivstoffe an den Orthodental-Brackets. Die unterschiedlichen Substanzen unterschiedlicher Körnung lagern sich ungewünscht ab, da sie mit der Zahnbürste nicht mehr entfernt werden können. Die Folge ist eine eingeschränkte Wirkung, da die ungewünschten Ablagerungen die Beweglichkeit des Archwires im Orthodental-Bracket reduzieren, was sich auf die Therapie störend auswirkt.

Um störende Ablagerungen auf und an bewegbaren Teilen von Orthodental-Brackets zu vermeiden, können Orthodentalpatienten auf abrasivstofffreie Zahnpasten zurückgreifen. Dann sind aber oftmals die Reinigungsergebnisse nicht ausreichend. Man hat auch versucht verschiedene zahnschonend reinigende, sich aber kaum störend ablagernde Abrasivstoffe zu verwenden.

Entsprechend der DE4008995 ist eine Zahnpaste bekannt, welche als Abrasivstoff Aktivkohlepulver enthält. Die Körnigkeit der Aktivkohle ist so gewählt, dass die Aktivkohlepartikel offenporig und feinkörnig sind und mittlere Durchmesser zwischen 2 und 20µm aufweisen, wobei die Oberflächenentwicklung bzw. die innere Oberfläche grösser als 300 m²/g ist. Die abrasive Wirkung der Aktivkohlepartikel in einer Formulierung zwischen 0.5 Gew.-% und 50 Gew.-% und erwähnter Körnung ist nicht ausreichend, sodass das Putzergebnis für Patienten mit Orthodental-Brackets nicht ausreichend ist. Zusätzlich kann es trotzdem zu störenden Ablagerungen an Zahnklammern aufgrund in der Zahnpaste weiterhin vorhandener Mineralien wie Kieselerde und verschiedenen Karbonaten kommen, ebenso wie die Ablagerungen der sehr kleinen Kohlepartikel selbst. Ob es sich bei den Aktivkohlepartikeln um Steinkohle oder Holzkohle handelt ist nicht offenbart.

Zur Vermeidung von Ablagerungen kann versucht werden zusätzlich Mundspülungen nach dem Zähneputzen zu verwenden. Entsprechend muss eine geeignete Mundspülung gefunden und angeschafft werden. Dabei kann nicht davon ausgegangen werden, dass die Ablagerungen beseitigt werden.

Aus der WO2009/045856 ist eine spezielle Zahnbürste bekannt, welche Borsten aufweist, in welche beispielsweise Aktivkohlepartikel eingelagert sind. Diese Aktivkohlepartikel werden beim Putzen der Zähne aus den Borsten freigesetzt und dienen als Aktivator für eine bleichende Reaktion mit einem in der verwendeten Zahnpaste vorhandenen Bleichmittel. Durch die Verwendung der Zahnbürste und der Zugabe von Aktivkohlepartikeln an die Zahnpaste, welche zwingend ein Bleichmittel enthalten muss, ist ein bleichender Effekt beim Zähneputzen erreichbar. Aktivkohle dient hier lediglich als Aktivator, wobei keine Angaben über die Form und Beschaffenheit der Aktivkohlepartikel gemacht werden. Gemäss der WO2009/045856 wird nicht auf die spezielle Problematik bei Patienten mit Zahnklammern eingegangen und die beschriebene Zahnbürste ist nicht für das mehrmals tägliche Zähneputzen gedacht.

Eine weitere Zahnbürste mit in den Borsten eingelagerten Aktivkohlepartikeln ist aus der WO 98 55 001 A1 bekannt.

### Darstellung der Erfindung

Die vorliegende Erfindung hat sich zur Aufgabe gestellt Patienten mit Orthodental-Apparaturen zur Korrektur von Kiefer- und Zahnfehlstellungen, beispielsweise von Orthodental-Brackets mit darin geführtem Archwire eine Möglichkeit zu schaffen, das tägliche Zähneputzen ohne das Auftreten störender Ansammlungen von Abrasivmittelpartikeln an den Orthodental-Apparaturen zu erreichen. Es soll trotz Tragen von Orthodental-Apparaturen, eine optimale Reinigung der Zahnoberflächen und Orthodental-Apparaturen gewährleistet sein, wobei die Mechanik störende Partikelansammlungen vermieden werden sollen.

Diese Aufgabe löst eine Zahnbürste in deren Borsten Aktivkohlepartikel ausreichender Grösse freisetzbar eingelagert sind.

### Beschreibung

Patienten mit zahnmedizinischen Apparaturen zur Korrektur von Kiefer- und Zahnfehlstellungen sollten abrasivstofffreie Zahnpasten benutzten. Dabei verzichtet die Zahnpaste auf den Zusatz von mineralischen körnigen Substanzen, die schleifend oder bleichend auf den Zahnschmelz wirken. Eine derartige Zahnpaste ist beispielsweise unter dem Markennamen Zahnschnee® des Anmelders bekannt. Weiterhin kann die abrasivmittelfreie Zahnpaste bekannte Schaumbildner, Feuchthaltemittel, Aroma- oder Geschmacksstoffe, sowie Konservierungsmittel, Farb- und Zusatzstoffe und prophylaktisch gegen Parodontose oder Karies wirkende Substanzen umfassen.

Aufgrund der fehlenden Abrasivstoffe in der Zahnpaste ist die Fähigkeit zur Beseitigung von Plaque und Bakterien von den Zahnoberflächen reduziert vorhanden. Abrasivstoffe werden in Form von Abrasivstoffpartikeln in die Borsten einer Zahnbürste eingelagert. Wie bekannt umfasst eine solche Zahnbürste einen Bürstenkopf an dem eine Mehrzahl von Borsten in unterschiedlichen Ausrichtungen, unterschiedlicher Härte und mit verschieden geformten Borstenenden angeordnet sein kann. Die Borsten können aus verschiedenen Kunstfasern, beispielsweise aus Polyamiden wie Nylon oder aus Polyester hergestellt sein. Materialien zur Herstellung unterschiedlich weicher Borsten sind dem Fachmann bekannt.

Das Borstenmaterial wird vor der Borstenherstellung mit Aktivkohlepartikeln mit mittleren Durchmessern von grösser gleich 100 µm gemischt, wobei ein Kunststoff/Aktivkohle-Gemisch mit Aktivkohlepartikeln resultiert. Die Aktivkohlepartikel werden in Form von Pulver aus Holzkohle in das Borstenmaterial eingefügt, damit eine möglichst homogene Verteilung erfolgt. Üblicherweise wird eine Masse des Kunststoffes mit zugesetzten Aktivkohlepartikeln erzeugt, welche später zu Granulat verarbeitet wird und zur weiteren Verarbeitung bereit ist.

Die Borsten werden im Kunststoffstrangguss hergestellt, wobei aus dem Kunststoff/Aktivkohle-Gemisch Granulat Borsten mit Durchmessern von etwa 150 µm bis 300 µm erzeugt werden, in welchen die Aktivkohlepartikel verteilt sind. Der Kunststoff bildet eine Matrix, in welcher die Aktivkohlepartikel mit einem mittleren Durchmesser von 100 µm eingelagert sind, wobei Aktivkohlepartikel an den Borstenköpfen durch entsprechende Bearbeitung freigelegt sind und teilweise aus dem Kunststoff herausragen können. Borsten mit einem Volumenanteil an Aktivkohlepartikeln von grösser gleich 40% stellen sicher, dass während des Gebrauchs und er Abnutzung der Borsten immer genügend Aktivkohlepartikel freigesetzt werden.

Der mittlere Durchmesser der verwendeten Aktivkohlepartikel sollte etwa 30% bis 70% des Durchmessers des Borstendurchmessers, bevorzugt etwa 50% des Borstendurchmessers betragen.

Die Aktivkohlepartikel umfassen offenporige feinkörnige Kohlepartikel aus Holzkohle mit einer inneren Oberfläche von 300 bis 2000 m²/g. Hier verwendbare Aktivkohlepartikel können nur auf pflanzlicher Basis sein, wobei reiner Kohlenstoff ohne Zusatz weiterer Chemikalien verwendet wird.

Neben einer abrasiven und reinigenden Wirkung, wirken die Aktivkohlepartikel entzündungshemmend, adsorbierend bleichend auf die Zahnoberfläche. Aufgrund der Einlagerung der Aktivkohlepartikel in die Kunstfaserborsten ist eine Überdosierung der Abrasivmittel ausgeschlossen. Die Verwendung von Holzkohle stellt eine äusserst milde Form von Abrasivmittel dar, wie es üblich für kratzfreies Polieren ist. Durch den Kontakt mit der Zahnpaste und der mechanischen Betätigung der Zahnbürste lösen sich Aktivkohlepartikel schrittweise und mit einer maximalen Rate, bei welcher keine störenden Abrasivmittelablagerungen erfolgen. Da die Abrasivmittel sich direkt auf den Zahnoberflächen aus den Borsten herauslösen, resultiert eine Reinigungswirkung direkt am Berührungspunkt der Borsten mit der Zahnoberfläche. Somit ist im Vergleich zur Benutzung aktivkohleenthaltener Zahnpasten ein gezielteres Aufbringen der Abrasivmittel möglich.

Bei der Verwendung einer abrasivstofffreien Zahnpaste mit der Zahnbürste, umfassend Borsten mit einem Anteil von grösser gleich 40 Volumenprozent Aktivkohlepartikel mit einem mittleren Durchmesser von grösser gleich 100µm, werden einzelne Aktivkohlepartikel während des Zähneputzens aus den Borsten herausgelöst und dienen als bleichende polierende Partikel und damit als Abrasivmittel auf der Zahnoberfläche.

Durch die Zuführung der Aktivkohlepartikel aus der Matrix der Borsten, gelangt eine ausreichend grosse Menge an Abrasivmittel an die Zahnoberfläche, wobei eine Verklumpung und Bildung von störenden Ablagerungen an orthodentalen Vorrichtungen ausgeschlossen ist.

Der Verkauf eines Systems aus einer abrasivstofffreien Zahnpaste und einer Zahnbürste mit entsprechend mit Aktivkohlepartikeln geeigneter Grösse und Verteilung versehenen Borsten, bieten sich für Patienten mit zahnmedizinischen Apparaturen zur Korrektur von Kieferund Zahnfehlstellungen, wie Orthodental-Brackets, an. Diese Patienten können bei Verwendung eines solchen Systems auf die Anschaffung zusätzlicher Mundspülungen verzichten und trotzdem eine Zahnoberflächenreinigung ohne den Verbleib von Zahnpastenrückständen an den Orthodental-Brackets erreichen.

## Patentansprüche

1. Zahnbürste umfassend Borsten aus Kunstfasern,
**dadurch gekennzeichnet, dass**
in die Kunstfasern Aktivkohlepartikel aus Holzkohle mit mittleren Durchmessern von grösser gleich 100 µm eingelagert sind, wobei der Anteil der Aktivkohlepartikel in den Borsten grösser gleich 40 Volumenprozent ist.

2. Zahnbürste nach Anspruch 1, **wobei** der mittlere Durchmesser der Aktivkohlepartikel aus Holzkohle etwa 30% bis 70% des Durchmessers der Borsten beträgt.

3. Zahnreinigungssystem zur Reinigung von Zahnoberflächen von Patienten mit Orthodental-Brackets,
**dadurch gekennzeichnet, dass** das System eine abrasivmittelfreie Zahnpaste sowie eine Zahnbürste gemäss Anspruch 1 umfasst.

4. Zahnreinigungssystem nach Anspruch 3, **wobei** der mittlere Durchmesser der Aktivkohlepartikel aus Holzkohle etwa 30% bis 70% des Durchmessers der Borsten beträgt.

## Claims

1. The toothbrush comprising bristles of synthetic fibers,
**characterized in that**
activated carbon particles from charcoal having average diameters greater than or equal to 100 µm are incorporated into the synthetic fibers, whereby the amount of activated carbon particles in the bristles is greater than or equal to 40 vol %.

2. The toothbrush according to claim 1, wherein the average diameter of the activated carbon particles from charcoal amounts to approximately 30% to 70% of the diameter of the bristles.

3. A dental cleaning system for cleaning the tooth surfaces of patients with orthodontic braces, **characterized in that**
the system comprises an non-abrasive toothpaste and a toothbrush according to claim 1.

4. The dental cleaning system according to claim 3, wherein the average diameter of the activated carbon particles form charcoal amounts to approyimately 30% to 70% of the diameter of the bristles.

## Revendications

1. Brosse à dents comprenant des poils en fibres synthétiques,
**caractérisée en ce que**
des particules de charbon actif de charbon de bois de diamètres moyens supérieurs ou égaux à 100 µm sont intercalées dans les fibres synthétiques, la part de particules de charbon actif dans les poils étant supérieure ou égale à 40 pour cent du volume.

2. Brosse à dents selon la revendication 1, le diamètre moyen des particules de charbon actif de charbon de bois étant environ 30% à 70% du diamètre des poils.

3. Système de nettoyage des dents pour le nettoyage des surfaces des dents des patients avec des supports orthodontiques,
**caractérisé en ce que** le système comprend un dentifrice dépourvu de moyen abrasif ainsi qu'une brosse à dents selon la revendication 1.

4. Système de nettoyage des dents selon la revendication 3, le diamètre moyen des particules de charbon actif de charbon de bois étant environ 30% à 70% du diamètre des poils.
